# EUROPEAN PATENT APPLICATION

(11) **EP 3 081 938 A1**
(43) Date of publication of application: **19.10.2016**
(21) Application number: 15164834.2
(22) Date of filing: 23.04.2015
(51) Int. Cl.: G01N 33/574, G01N 33/92

(54) **SERUM BIOMARKER FOR HEPATOCELLULAR CARCINOMA (HCC)**

(30) Priority: 13.04.2015 EP 15001051
(71) Applicant: Johann Wolfgang Goethe-Universität Frankfurt am Main, 60323 Frankfurt am Main (DE)
(72) Inventor: Grammatikos, Georgios, 60594 Frankfurt am Main (DE); Pfeilschifter, Josef, 60598 Frankfurt am Main (DE); Zeuzem, Stefan, 60323 Frankfurt am Main (DE); Ferreirós, Nerea, 60596 Frankfurt am Main (DE)
(74) Representative: Krauss, Jan

(57) **Abstract**

The present invention pertains to novel diagnostic procedures for the prognosis, diagnosis and monitoring of liver diseases such as hepatocellular carcinoma (HCC). The present invention provides sphingolipids and especially long chain ceramides as significant and highly prognostic serum biomarkers in HCC compared to liver cirrhosis. Therefore the invention in particular provides a method for detecting the presence or absence of a HCC in a liver cirrhosis patient using the disclosed biomarkers. Also provided is a method for monitoring the treatment success of a HCC treatment by monitoring the disclosed serum biomarkers. Finally the invention pertains to diagnostic kits for performing the disclosed methods of the invention.

## Description

### FIELD OF THE INVENTION

The present invention pertains to novel diagnostic procedures for the prognosis, diagnosis and monitoring of liver diseases such as hepatocellular carcinoma (HCC). The present invention provides sphingolipids and especially long chain ceramides as significant and highly prognostic serum biomarkers in HCC compared to liver cirrhosis. Therefore the invention in particular provides a method for detecting the presence or absence of a HCC in a liver cirrhosis patient using the disclosed biomarkers. Also provided is a method for monitoring the treatment success of a HCC treatment by monitoring the disclosed serum biomarkers. Finally the invention pertains to diagnostic kits for performing the disclosed methods of the invention.

### DESCRIPTION

Hepatocellular carcinoma (HCC) constitutes a major health burden since it represents the sixth more common cancer and the third leading cause of cancer related mortality worldwide. In most cases HCC occurs within a background of liver cirrhosis being the most frequent cause of death in these patients. This urges both basic and translational research approaches to improve current diagnostic tools for early detection and surveillance of HCC. Moreover, lack of treatment options for advanced HCC and shortness of available liver allografts for potentially curable patients render the identification of novel target pathways and biomarkers an eminent research goal.

HCC is often associated with chronic liver injury or disease. Cirrhosis of any etiology is the most common risk factor for HCC development. Over 90% of HCCs develop on a cirrhotic liver resulting from either chronic hepatitis B virus (HBV) or hepatitis C virus (HCV) infections, alcohol abuse, or accumulation of fat referred to as non-alcoholic steatohepatitis (NASH). Accordingly, there is a need for an assay to detect HCC prior to development of clinical symptoms in "at-risk" patients, i.e., those with pre-existing liver conditions such as cirrhosis. A- fetoprotein (AFP) is widely used as a surveillance and detection test for hepatocellular carcinoma (HCC) among patients with cirrhosis. However, AFP is not effective for detection of early stage HCC (Sanyal et al, Oncologist 15): 14-22, 2010; Bruix and Sherman, Hepatology 42: 1208-1236, 2005; Di Bisceglie, Gastroenterology 127:S104-S107, 2004; Daniele et al, Gastroenterology 127:S108-S112, 2004). Apart from AFP, lectin-bound AFP (AFP-L3), des-gamma carboxyprothrombin (DCP) and glypican-3 have been proposed as markers for HCC detection (Spangenberg et al, Semin. Liver Dis. 26:385-390, 2006). However, a recent study showed that neither DCP nor AFP-L3 presented better performance characteristics than AFP for the diagnosis of early stage HCC (Marrero et al., Gastroenterology 137: 110-118, 2009) and that neither DCP nor AFP is optimal to complement ultrasound in the detection of early HCC (Lok et al, Gastroenterology 138:493-502, 2010). Therefore, there is a need for the development of novel biomarkers and assays for the early detection and prediction of a subject's risk of developing HCC to allow for early intervention.

Targeting sphingolipid (SL) metabolism in cancer has gained significant attention in the last two decades since both proliferation and apoptosis of tumors as well as cancer drug resistance are substantially regulated by SL's. Ceramide (Cer), the bioactive hydrophobic backbone of various complex SL's, has been proposed by many studies as a potent tumor-suppressor molecule activated by common cancer treatment modalities such as chemotherapy and irradiation while abrogation of ceramide generation is observed in tumor tissues resistant to therapy.

The improved implementation of spectrometric methods in the last years has enabled a thorough study of SL metabolite variations in various diseases. Serum or plasma Cer's constitute in the meantime evident disease biomarkers in obesity and diabetes mellitus (Haus JM, et al. Plasma ceramides are elevated in obese subjects with type 2 diabetes and correlate with the severity of insulin resistance. Diabetes 2009;58:337-343), in acute phase reactions (Lightle S, et al. Elevation of ceramide in serum lipoproteins during acute phase response in humans and mice: role of serine-palmitoyl transferase. Arch Biochem Biophys 2003;419:120-128), in renal (Mitsnefes M, et al., Ceramides and cardiac function in children with chronic kidney disease. Pediatr Nephrol 2014;29:415-422) and neurodegenerative (Mielke MM, et al. Serum ceramides increase the risk of Alzheimer disease: the Women's Health and Aging Study II. Neurology 2012;79:633-641) disorders, while SIP is implicated as a biomarker in various cancer diseases (Nunes J, et al. Circulating sphingosine-1-phosphate and erythrocyte sphingosine kinase-1 activity as novel biomarkers for early prostate cancer detection. Br J Cancer 2012;106:909-915).

Despite the fact that several studies observed an implication of Cer and SIP as major regulators of hepatocellular susceptibility to various stimuli as well as of hepatocarcinogenesis both in vitro and in the mouse model, no data are available to date regarding SL's as biomarkers of HCC.

In view of the state of the art it was therefore an object of the present invention to provide new diagnostic biomarkers for the detection of HCC in patients suffering from liver cirrhosis.

The above problem is solved in a first aspect by a non-invasive method of predicting, monitoring and/or diagnosing a liver disease in a subject, comprising determining the level of one or more biomarkers selected from the group of sphingolipids in a biological sample from the subject.

In some embodiments the method may comprise the steps of:
(a) Providing the biological sample from the subject,
(b) Determining the level of one or more biomarkers selected from the group of sphingolipids in the biological sample;
(c) Comparing the level of the one or more biomarkers in the biological sample to a control level of the one or more biomarkers;
Wherein a different level of the biomarker in the biological sample compared to the control level is indicative for the presence or absence of a liver disease in the subject.

A "different level" in context of the invention shall refer to either an increased or decreased level. For example in some embodiments an increased level may be indicative for the presence of a liver disease. Then a decreased or equal level would be indicative for the absence of the liver disease. In other embodiments an equal or increased level may be indicative for the presence of a liver disease. Then a decrease of the level would be indicative for the absence of the liver disease.

The term "biological sample" means any biological sample derived from a subject/patient. Examples of such samples include tissues, cell samples, cell lysates, biopsies, etc. Biological samples may be selected from a tumor sample or a biopsy such as liver tumor sample. Other biological samples are whole blood, serum or plasma. Preferably, the sample is a whole blood sample. Most preferred in context of the present invention is that the biological sample is blood sample, such as a serum sample.

A "biomarker" or "marker" in the context of the present invention refers to an organic bio-molecule, particularly a metabolite such as a sphingolipid, which is differentially present in a biological sample taken from subjects having a certain condition as compared to a comparable sample taken from subjects who do not have said condition (e.g., negative diagnosis, normal or healthy subject, or non-cancer patients, depending on whether the patient is tested for cancer). For examples, a marker can be a polypeptide or lipid (having a particular apparent molecular weight) which is present at an elevated or decreased level in samples of cancer patients compared to samples of patients with a negative diagnosis. Negative diagnosis does not exclude the presence of other related diseases such as a negative diagnosis of HCC in a subject having liver cirrhosis.

The term "determining" as used herein includes qualitative and/or quantitative detection (i.e. detecting and/or measuring expression level) with or without reference to a control or a predetermined value. "Determining the level" shall refer to a quantitative detection of a biomarker as disclosed herein. For determining the level of sphingolipid in the biological sample and/or control sample any method can be used that allows the quantification of sphingolipid concentrations. In a preferred embodiment the content of sphingolipids in a sample to be analyzed is determined by tandem liquid chromatography mass spectrometry (LC-MS/MS), immunologically using a sphingolipid-specific antibody or by thin layer chromatography.

In certain instances, the methods of the invention are used in order to diagnose a liver disease, diagnosing HCC or diagnosing HCC in subject having liver cirrhosis. The methods can be used to monitor the disease, both progression and regression. The term "monitoring" therefore may refer both to "monitoring the progression or regression of HCC or liver cirrhosis" and includes the use of the methods and biomarker profiles to determine the disease state (e.g., presence or severity of HCC) of a subject. In certain instances, the results of a statistical analysis are compared to those results obtained for the same subject at an earlier time.

In some aspects, the herein disclosed biomarkers are used as prognostic (predicting) biomarkers in accordance with the disclosed methods. In this respect the methods of the present invention can also be used to predict the progression of a liver disease, e.g., by determining a likelihood for a liver disease to progress either rapidly or slowly in an individual based on the presence or level of at least one biomarker in a sample. In other aspects, the methods of the present invention can also be used to predict the regression of a liver disease, e.g., by determining a likelihood for a liver disease to regress either rapidly or slowly in an subject based on the presence or level of at least one biomarker in a biological sample in accordance with the present disclosure.

In some embodiments of the invention the liver disease is liver cirrhosis or liver cancer, preferably hepatocellular carcinoma (HCC). As used herein, the term "hepatocellular carcinoma" (or "HCC" for short) refers to a malignant tumor of hepatocellular origin. HCC is a type of liver cancer.

The term "subject," as used herein, describes a mammal including, but not limited to, humans, apes, chimpanzees, orangutans, monkeys, dogs, cats, horses, pigs, sheep, goats, mice, rats, and guinea pigs. However, in some embodiments a subject is preferably a human. In certain embodiments, where the methods of the present invention are used for distinguishing a HCC from liver cirrhosis, the subject is a subject suffering from liver cirrhosis, but potentially also having HCC. Distinguishing liver cirrhosis from HCC shall mean in context of the present invention that HCC is diagnosed in a cirrhotic background, in other words in subjects having liver cirrhosis.

In some preferred embodiments the herein disclosed methods are strictly performed *in vitro* or *ex vivo.* In particular the term "providing a biological sample from the subject" should not be misinterpreted to explicitly or implicitly include an invasive method step of obtaining the biological sample from a living subject. The term "providing a biological sample" shall explicitly exclude any steps of obtaining a biological sample from a subject.

The term "level" in conjunction with one or more biomarkers of the present disclosure shall refer preferably to the concentration of the respective biomarker.

In some embodiments of the present invention the one or more biomarker is preferably selected from a long chain ceramide, or a long chain dihydroceramide, preferably a C16 to C24 Ceramide or C16 to C24 dihydroceramide. More preferably the biomarker is a C16 to C20 ceramide or C16 to C20 dihydroceramide. For example the one or more biomarker may be a sphingolipid selected from the group of C16Cer, C16DHC, C18DHC, SIP, C24DHC, C24:1DHC, C18Cer, C20Cer, C24Cer, C24:1Cer, sphingosine, and SA1P. In most preferred embodiments of the invention the one or more biomarker is selected from the group C16Cer, C16DHC, C18DHC, and SIP, and preferably is C16Cer.

It was particularly surprising that although all of the sphingolipid biomarkers of the invention are of diagnostic value, the biomarkers C16DHC, C18DHC, C16Cer and SIP showed an even better prognostic value as determined by AUC analysis than the currently state of the art marker for HCC detection AFP.

A ceramide includes a sphingosine bound to a fatty acid via an amide linkage. Where the term "CnCer", "Cn ceramide" or "CnDHC", "Cn dihydroceramide" is used, n is an integer and refers to the number of carbons (C) in the fatty acid residue, e.g., C16Cer refers to a ceramide core having a 16-carbon fatty acid residue, such as palmitoyl, and C18Cer refers to a ceramide core having a 18- carbon fatty acid residue, such as stearoyl.

Other embodiments of the present invention then provide the herein described methods wherein step (b) comprises determining the level of a least one additional biomarker selected from the group of C16Cer, C16DHC, C18DHC, SIP, C24DHC, C24:1DHC, C18Cer, C20Cer, C24Cer, C24:1Cer, Sphingosine, and SA1P, wherein the first and the additional biomarker and not the same.

Yet another embodiment of the invention pertains to the methods of the invention wherein diagnosing a liver disease comprises diagnosing in a subject having liver cirrhosis, the presence or absence of HCC. Cirrhosis is a major factor in the development of HCC and therefore HCC patients often have a history of liver cirrhosis. Therefore it is advantageous to monitor cirrhotic patients constantly for the occurrence of HCC. Detecting the HCC early greatly improves prognosis and treatment success of the patient. Such a monitoring is now possible using the biomarkers of the present invention, preferably according to the herein described methods.

The term "control level" as used in the context of the present invention may refer to various reference values depending on the diagnostic context for which the present method is used. A control level may therefore be any reference value of the respective biomarker which allows for a meaningful interpretation of the status or development of a liver disease in a patient. In general a control level may correspond to a level of the biomarker in a biological sample of subjects not having the liver disease. Alternatively a control level may correspond to a level of the biomarker in a biological sample of the subject at an earlier time point, for example before said subject underwent a therapy or medical treatment. In certain embodiments of the invention the control level is a cut-off level, and an increased level or equal level of the one or more biomarker in the biological sample compared to the cut-off level is indicative for the presence of the liver disease. Most preferably an increased level or equal level of the one or more biomarker in the biological sample compared to the cut-off level is indicative for the presence of HCC in the subject.

A cut-off level of the one or more biomarker of the present invention is preferably selected such that in a receiver operating characteristics (ROC) analysis at 95% confidence interval the area under the curve (AUC) is at least 0.6, more preferably 0.7, 0.8, and most preferably at least 0.9, 0.95 or higher.

According to the examples, preferred cut-off levels of the various biomarkers of the present invention are
(i) For C16DHC about 55.2 ng/ml,
(ii) For C18DHC about 64.2 ng/ml,
(iii) For C24DHC about 65.1 ng/ml,
(iv) For C24:1DHC about 301 ng/ml,
(v) For C16Cer about 147.5 ng/ml,
(vi) For C18Cer about 67.7 ng/ml,
(vii) For C20Cer about 78.6 ng/ml,
(viii) For C24Cer about 953 ng/ml,
(ix) For C24:1Cer about 431 ng/ml,
(x) For sphingosine about 4.83 ng/ml,
(xi) For S1P about 215 ng/ml, and
(xii) For SA1P about 30.8 ng/ml.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 20%, 15%, 10%, 5%, 1%, or 0.5% of a given value or range. The above cut-off values are preferably in the range of +/- 15% of the mentioned numerical values. Alternatively, the term "about" means within an acceptable standard error of the mean when considered by one of ordinary skill in the art. Unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

Since the biomarkers of the present invention are of highly prognostic value for the detection of HCC, alternative embodiments of the invention pertain to methods wherein the control level is level of the one or more biomarkers in a healthy subject not having a liver disease, and an increased level of the one or more biomarkers in the biological sample compared to the control level indicates the presence of the liver disease in the subject. In this embodiment the method of the invention provides information about the presence or absence of a liver disease in a subject. Preferably the liver disease is HCC.

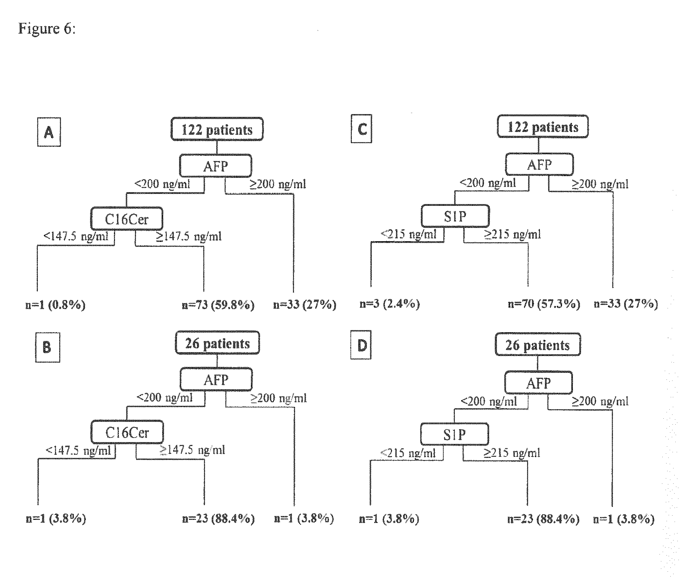

Some embodiments of the present invention provide the aforementioned methods further comprising the use of AFP as an additional biomarker. AFP is currently state of the art biomarker for the detection of HCC in cirrhotic patients. Therefore, the invention provides diagnostic method wherein a biological sample of a subject is for example first tested for the biomarker AFP according to state of the art methods and then further tested with a diagnostic method as described herein before, or *vice versa*. In particular preferred are diagnostic algorithms as described in figure 6 and the corresponding examples.

The above problem of the invention is furthermore solved in another aspect by a non-invasive method for monitoring a treatment of a liver disease in a subject, the method comprising
(a) Providing a first biological sample of the subject that was taken at a first time point of the therapy,
(b) Providing a second biological sample of the subject that was taken at a second time point of the therapy, wherein the second time point is later that the first time point,
(c) Determining the level of one or more biomarker selected from the group of sphingolipids in the first and the second biological sample,
(d) Comparing the level of the one or more biomarker in the first biological sample with the level of the one or more biomarker in the second biological sample,
Wherein an increase of the level of the one or more biomarker in the second biological sample compared to the first biological sample is indicative for a treatment success. As already described herein before, the method is preferably an *in vitro* or *ex vivo* method. The same general descriptions regarding the non-invasive character of the methods of the invention applies similarly to this aspect.

The one or more biomarker may be selected from the group of C16Cer, C16DHC, C18DHC, SIP, C24DHC, C24:1DHC, C18Cer, C20Cer, C24Cer, C24:1Cer, Sphingosine, and SA1P.

Yet another aspect of the invention pertains to a diagnostic kit for performing a method in accordance with the present discloure, wherein the kit comprises means for determining the level of the one or more biomarkers in the biological sample.

The present invention will now be further described in the following examples with reference to the accompanying figures and sequences, nevertheless, without being limited thereto. For the purposes of the present invention, all references as cited herein are incorporated by reference in their entireties. In the Figures:

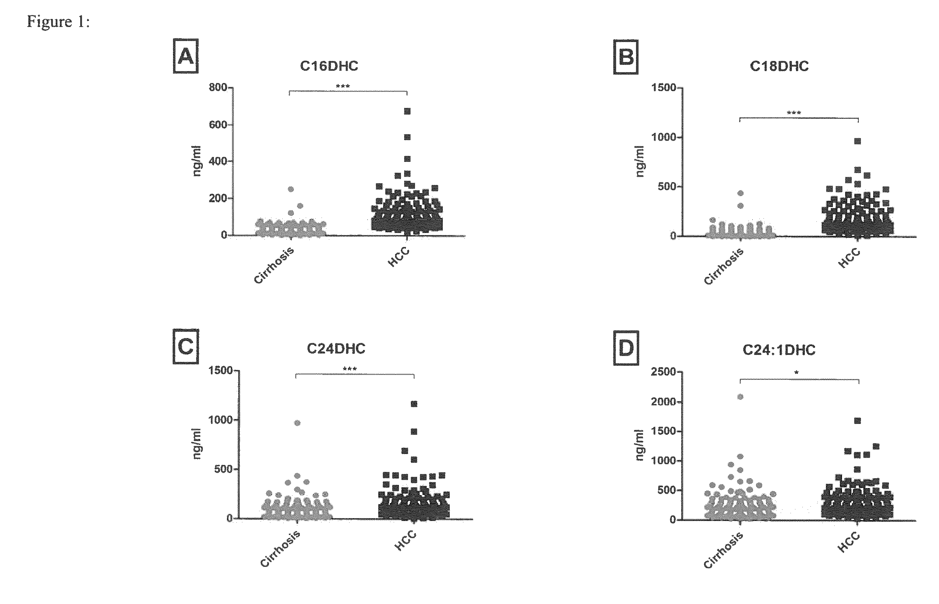

- **Figure 1:**: Serum dihydroceramides are upregulated in HCC patients: Both long chain DHC's (C16DHC and C18DHC) as well as very long chain DHC's (C24DHC and C24:1DHC) show significantly higher concentrations in the serum of HCC patients as compared to patients with liver cirrhosis (P<0.001 for C16DHC, C18DHC, C24DHC and P<0.05 for C24:1DHC). DHC: dihydroceramide.

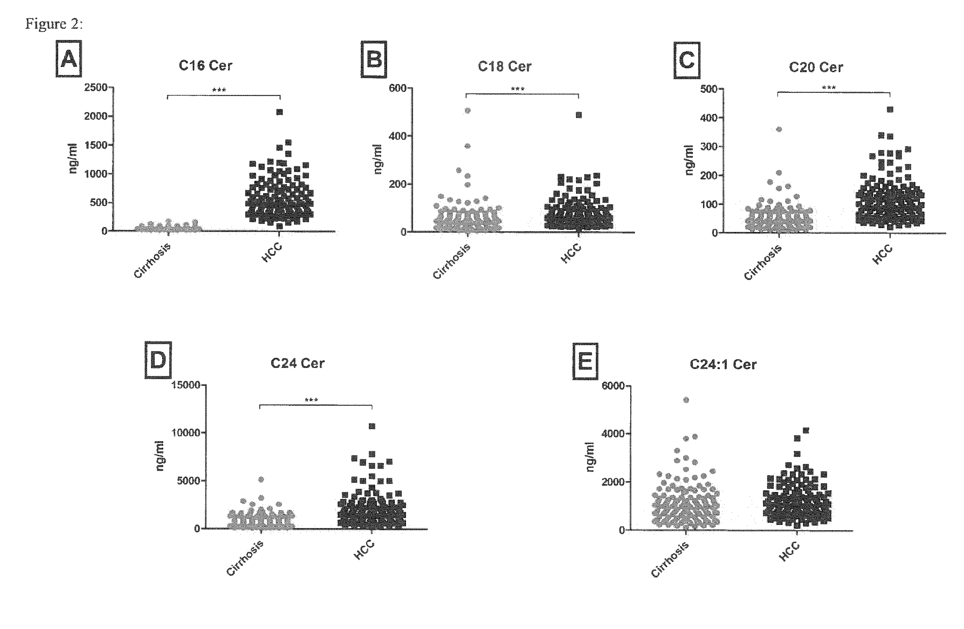

- **Figure 2:**: Ceramides accumulate in the serum of patients with HCC: Except for the unsaturated derivative of C24Cer, C24:1Cer (2E), all serum Cer's assessed were upregulated in the serum of HCC patients as compared to patients with liver cirrhosis (P<0.001). Cer: ceramide.

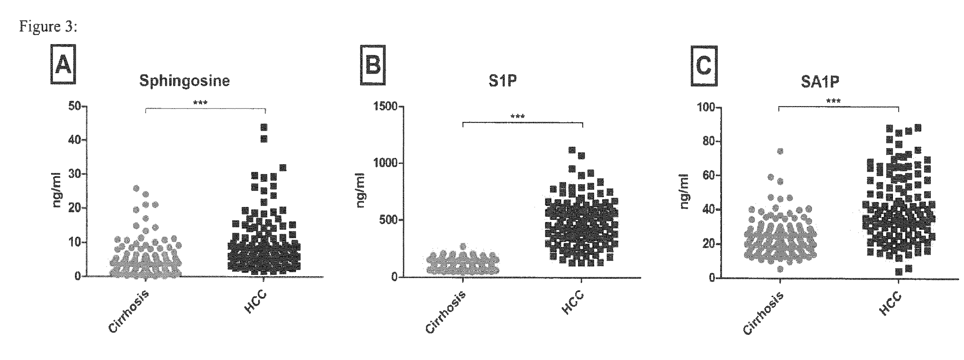

- **Figure 3:**: Sphingosine, SIP and SA1P in HCC patients: Serum sphingosine, SIP and SA1P are highly elevated in HCC patients as compared to patients with liver cirrhosis (P<0.001). SIP: sphingosine 1-phosphate, SA1P: sphinganine 1 - phosphate.

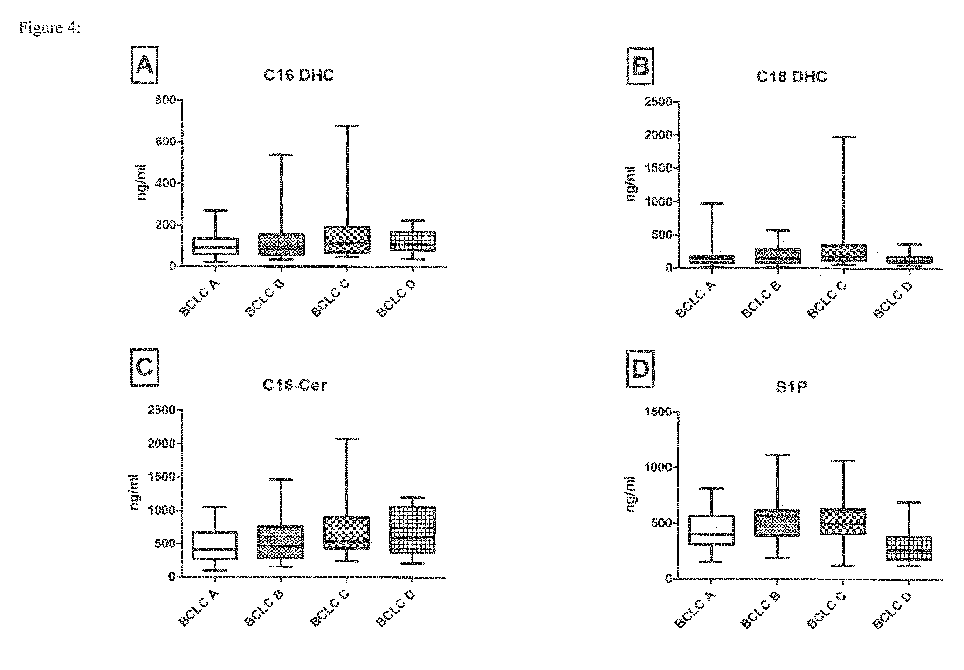

- **Figure 4:**: Serum SL parameters and BCLC stage: No significant variations between serum concentrations of various SL parameters and BCLC stage of HCC were observed in this study. SL: sphingolipid, BCLC: Barcelona Clinic Liver Cancer, HCC: hepatocellular carcinoma, Cer: ceramide, DHC: dihydroceramide, SIP: sphingosine 1-phosphate.

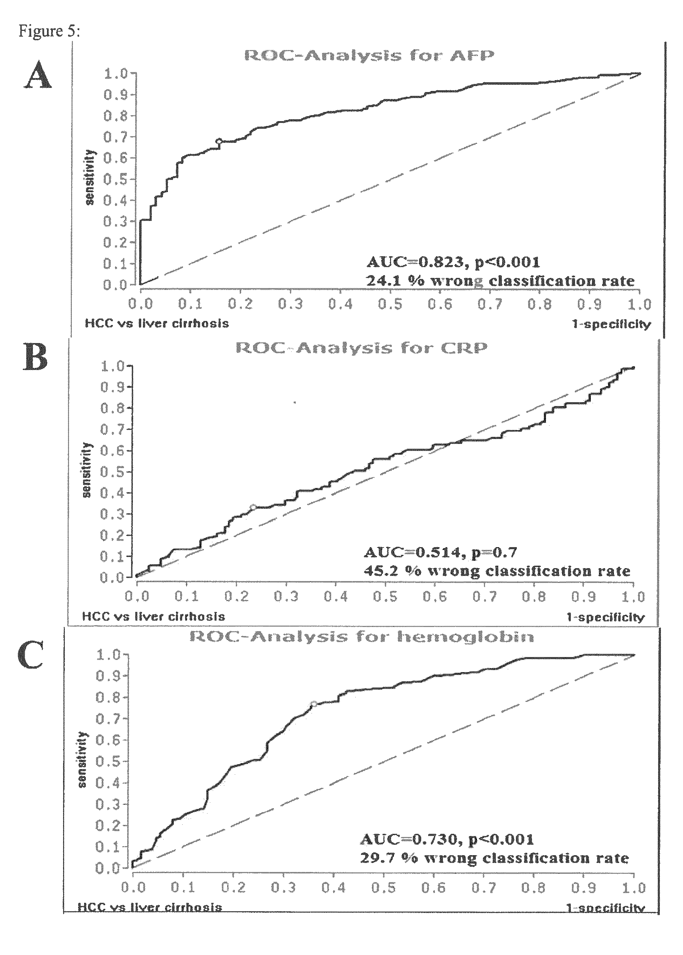

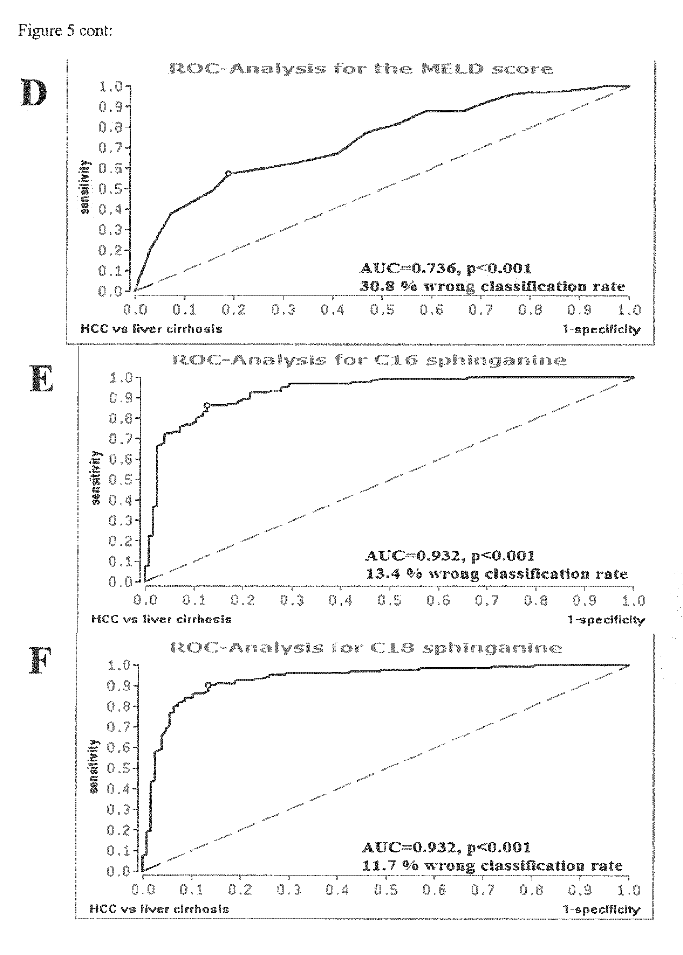

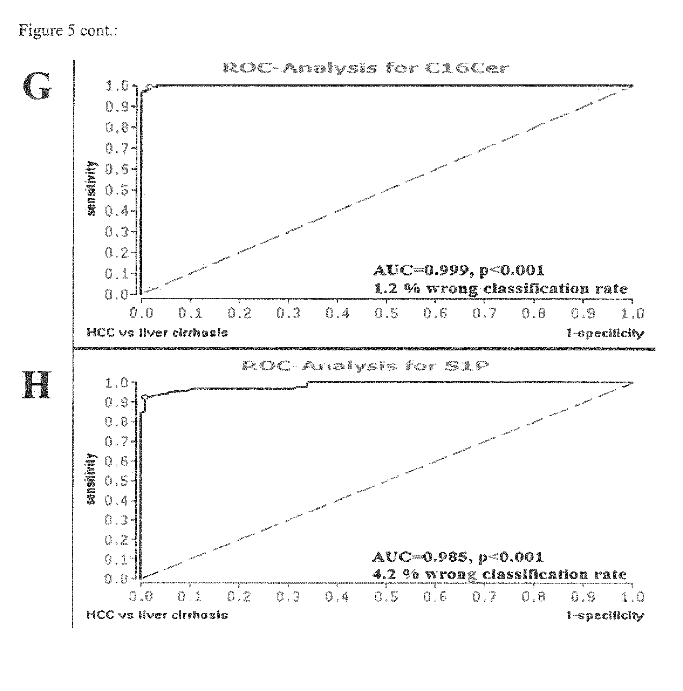

- **Figure 5:**: Diagnostic performance of serum sphingolipids and further biochemical parameters in the differentiation of HCC from liver cirrhosis: ROC analysis identified serum SL parameters (4E-4H) with a superior diagnostic accuracy as compared to common biochemical markers as well as to AFP (4A-4D), the only widely available serologic marker of HCC. ROC: receiver operating curve, SL: sphingolipid, AFP: alpha fetoprotein, AUC: area under the curve, HCC: hepatocellular carcinoma.
- **Figure 6:**: Diagnostic algorithm of non-invasive diagnosis of HCC by C16Cer or SIP: Implementation of the diagnostic algorithm of non-invasive HCC diagnosis according to AFP, C16Cer or SIP in all HCC patients (A, C) and in patients with an early stage BCLC A HCC (B, D). HCC: hepatocellular carcinoma, Cer: ceramide, SIP: sphingosine 1-phosphate, BCLC: Barcelona Clinic Liver Cancer.

### EXAMPLES

### Materials and Methods

### HCC patients

Serum samples of 122 patients with HCC who were followed between February 2009 and April 2013 at the Department of Internal Medicine of the Johann Wolfgang Goethe University Hospital Frankfurt, Germany were routinely stored and used for the present study. Patients were included in a previously published prospective cohort. The diagnosis of HCC was made according to the EASL practice guidelines by histopathology or by dynamic imaging with characteristic hypervascularity in the arterial phase and washout in the portal venous phase. Exclusion criteria were an age below 18, history of cancer other than HCC in the last five years, history of solid organ transplantation and local or systemic treatment for HCC within the last 28 days. The diagnostic potential of SL parameters was assessed at the day of study inclusion. The Barcelona Clinic Liver Cancer (BCLC) stage, model of end stage liver disease (MELD) score and Child Pugh stage were assessed by clinical examination, laboratory parameters and the results of abdominal ultrasound examination, computed tomography or magnetic resonance imaging at the time of inclusion in the study.

### Patients with liver cirrhosis

An age- and gender-matched control group of 127 patients with comparable liver function was derived from a previously published cohort of patients with liver cirrhosis who were treated and initially participated from March 2009 until June 2011 in a prospective cohort study. From this cohort, patients were selected for the present study as follows: For each HCC patient included in the present study, 1 patient with liver cirrhosis was randomly matched according to age and sex. Before matching, both patient cohorts were stratified in groups according to age (18-29, 30-39, 40-49, 50-59, and >60). Within these groups, patients were randomly matched for sex. Randomization was performed based on a numerical order of a random identification number, which had been assigned in the original prospective study in which all patients with liver cirrhosis had been included. Inclusion criteria were liver cirrhosis, proven by histopathological examination of liver biopsy material or explicit morphological criteria of liver cirrhosis in ultrasound, computed tomography or magnetic resonance imaging and an age ≥18 years. Exclusion criteria were a history of malignant disease within the last five years and former solid organ or bone marrow transplantation.

### Determination of SL Concentrations by High- Performance Liquid Chromatography/Tandem Mass Spectrometry

Quantification of serum SLs was performed by high-performance liquid chromatography/tandem mass spectrometry (LC-MS/MS), as previously described (Grammatikos G, et al. Variations in serum sphingolipid levels associate with liver fibrosis progression and poor treatment outcome in hepatitis C virus but not hepatitis B virus infection. Hepatology 2015;61:812-22). For quantitation of SLs, 20 mL of serum were extracted with methanol/chloroform/HCl (15:83:2).

Afterwards, amounts of C16:0Cer, C18:0Cer, C20:0Cer, C24:1Cer, C24:0Cer, C16:0dhCer, C18:0dhCer, C24:0dhCer, C24:1dhCer, and the internal standard (C17:0Cer) and sphingosine, SIP and SA1P, and the internal standards (sphingosine-D7, sphinganine-D7 and sphingosine 1-phosphate-D7) were analyzed by LC-MS/ MS. All serum samples were stored at - 80°C until assayed.

### Statistical Analysis

Statistical analysis for the scatter and box plots presented was performed with GraphPad Prism for Windows (v5.01; GraphPad Software Inc., San Diego, CA). Further statistical calculations were performed by using BiAS software for Windows (version 10.11; Epsilon-Verlag, Darmstadt, Germany). Statistical comparisons for continuous variables were carried out using the nonparametric Mann-Whitney's U and Kruskal-Wallis' tests to determine differences among patient groups. Dichotomic variables were assessed by means of contingency tables (Mantel-Haenszel's test), as appropriate. The data are expressed as means ± standard error, unless otherwise specified. The level of significance was set at a=0.05, representing the 95% confidence interval (CI). Statistically significant differences are indicated in the corresponding figures (*P<0.05; **P<0.01; ***P<0.001). The correlation coefficient rho was calculated by using the Spearman correlation provided in BiAS software. Receiver operating characteristic (ROC) curves and area under the curve (AUC) values were calculated by the BiAS software as well.

### Patient characteristics

A total of 249 patients with liver cirrhosis were included in the present study according to the above described criteria. 122 patients were diagnosed with HCC and variations of serum SL's were assessed and compared to an age- and sex-matched series of 127 patients with liver cirrhosis without HCC as described above. Baseline characteristics of the included patients are shown in Table 1. In order to exclude variations of serum SL's resulting from complications or therapy of liver cirrhosis and HCC the analysis was concentrated on serum samples obtained at the day of study inclusion in two previous prospective study cohorts. Chronic alcohol abuse, chronic HCV and chronic hepatitis B virus (HBV) infection were the major etiologies of chronic liver disease in both patient groups (Table 1).

**Table 1 Demographic, biochemical and clinical characteristics in patients with liver cirrhosis and in patients with HCC**

| **Parameters** | **Patients with liver cirrhosis and HCC** | **Patients with liver cirrhosis without HCC** | **P-value** |
|---|---|---|---|
| | **(n=122)** | **(n=127)** | |
| **Epidemiologic characteristics** | | | |
| Age, years | | | |
| Median (range) | *66 (39-87)* | *62(39-79)* | 0.047 |
| Sex | | | |
| Female, n= (%) | 19 (15.5) | 31 (24.4) | 0.082 |
| Male, n= (%) | 103 (84.4) | 96 (75.5) | |

| **Biochemical parameters** | | | |
|---|---|---|---|
| ALT, IU/l | | | |
| Median (range) | 48 (8-309) | 27 (6-1268) | <0.001 |
| AST, IU/l | | | |
| Median (range) | 80 (20-535) | 47 (15-2823) | <0.001 |
| γGT, IU/l | | | |
| Median (range) | 179 (20-1881) | 83 (14-1178) | <0.001 |
| Bilirubin, mg/dl | | | |
| Median (range) | 1.1 (0.2-20) | 1.7 (0.3-15) | <0.001 |
| Creatinine, mg/dl | | | |
| Median (range) | 0.89 (0.37-5.2) | 1.03 (0.42-5.02) | 0.001 |
| ALP, IU/l | 142 (11-937) | 113.5 (34-422) | 0.001 |
| Median (range) | | | |
| Albumin, g/dl | 3.65 (2.1-5.3) | 3.2 (1.7-5.2) | 0.001 |
| Median (range) | | | |
| CRP, mg/dl | 0.93 (0.03-17.4) | 1.19 (0.04-16.84) | 0.7 |
| Median (range) | | | |
| Platelets, /µl | 152 (22-485) | 98 (17-396) | <0.001 |
| Median (range) | | | |
| Hemoglobin, mg/dl | 12.8 (8.2-16.9) | 10.4 (7.0-16.0) | <0.001 |
| Median (range) | | | |
| INR | 1.17 (0.87-3.03) | 1.34 (0.90-2.76) | <0.001 |
| Median (range) | | | |
| AFP, ng/ml | 24.25 (1.4-65000) | 3.5 (1.3-210.3) | <0.001 |
| Median (range) | | | |

| **Etiology of liver disease** | | | |
|---|---|---|---|
| Alcohol abuse, n (%) | 37 (30.3) | 69 (54.3) | 0.014 |
| Hepatitis C, n (%) | 40 (32.7) | 34 (26.7) | 0.4 |
| Hepatitis B, n (%) | 23 (18.8) | 15 (11.8) | 0.1 |
| NASH, n (%) | 12 (9.8) | 2 (1.5) | 0.007 |
| Cryptogenic, n (%) | 7 (5.7) | 11 (8.6) | 0.4 |
| Other*, n (%) | 16 (13.1) | 11 (8.6) | 0.3 |

| **Severity of liver disease** | | | |
|---|---|---|---|
| MELD | 10 (6-25) | 14 (6-35) | <0.001 |
| Median (range) | | | |
| Child-Pugh-stage | | | |
| A, n (%) | 66 (54.0) | 24 (18.8) | <0.001 |
| B, n (%) | 34 (27.8) | 67 (52.7) | 0.009 |
| C, n (%) | 13 (10.6) | 35 (27.5) | 0.005 |

| **Severity of HCC** | | | |
|---|---|---|---|
| BCLC-stage | | | |
| A, n (%) | 26 (21.3) | - | - |
| B, n (%) | 45 (36.8) | - | - |
| C, n (%) | 32 (26.2) | - | - |
| D, n (%) | 14 (11.4) | - | - |

**Median with range or number of patients with percent in brackets.** Abbreviations: *HCC: hepatocellular carcinoma*, *ALT: alanine aminotransferase, AST: aspartate aminotransferase, γGT: gamma-glutamyl-transferase, ALP: alkaline phosphatase, CRP: C-reactive protein, INR: international normalized ratio, AFP: alpha-fetoprotein, NASH: non-alcoholic steatohepatitis, MELD: Model of End stage Liver Disease, BCLC; Barcelona Clinic Liver Cancer*.

### Example 1: Serum long and very long chain (dihydro-)ceramides as well as SA1P and SIP are upregulated in HCC

Serum concentrations of long (C16-C20) and very long (≥C24) chain Cer's as well as of their synthetic precursors, dihydroceramides (DHC's), were assessed in patients with HCC and in patients with liver cirrhosis. An accumulation of both DHC's (Figure 1) and of Cer's (Figure 2) in HCC as compared to liver cirrhosis was observed.

Except from C24:1Cer and C24:1DHC, the respective unsaturated derivatives of C24Cer and C24DHC, all Cer's and DHC's analyzed showed a highly significant accumulation in HCC as compared to liver cirrhosis (Figures 1A, B, C and 2A, B, C and D, P<0.001). Sphingosine, the bioactive amino-alcohol backbone of SL's, was upregulated in the serum of HCC patients as well (Figure 3A, P<0.001). Both its phosphate derivative SIP as well as the phosphate derivative of sphinganine, sphinganine 1-phosphate (SA1P) showed a significant accumulation in HCC as compared to liver cirrhosis (Figure 3B, 3C, P<0.001, respectively).

### Example 2: Correlations of sphingolipid parameters with biochemical and surrogate markers of HCC

Since SL parameters showed a significant accumulation in the serum of HCC patients Spearman rank correlations were used to identify potential relationships to demographic characteristics and biochemical parameters in these patients. Significant correlations of various DHC's and Cer's with markers of hepatocellular injury were identified, while none of the parameters assessed, correlated significantly with the age of the included patients (Table 2). Regarding the severity of liver disease, solely C16DHC and C16Cer showed a positive correlation with the MELD score (Table 2, P<0.05 and P<0.001 respectively) whereas levels of C20Cer, C24Cer, SIP and SA1P were inverse proportional to the MELD score (Table 2, P<0.01, P<0.001, P<0.001 and P<0.05 respectively). Interestingly, despite the fact that all of the SL parameters assessed showed an accumulation in the serum of HCC patients (Figures 1, 2 and 3), only a part of them associated with alpha fetoprotein (AFP), the only broadly available HCC biomarker in the clinical setting to date. Particularly, C16DHC, C18DHC, C24:1DHC, C16Cer, C18Cer and sphingosine correlated significantly with the levels of AFP (Table 2, P<0.01, P<0.001, P<0.01, P<0.001, P<0.05 and P<0.01 respectively).

**Table 2 Correlation of serum SL's with age, MELD score and biochemical parameters in HCC patients**

| **SL** | **Age** | **AST** | **ALT** | **γGT** | **AFP** | **CRP** | **Hb** | **MELD** |
|---|---|---|---|---|---|---|---|---|
| C16DHC | 0.065 | **0.409***** | **0.317***** | **0.406***** | **0.294**** | 0.155 | -**0**.**21*** | **0**.**208*** |
| C18DHC | 0.096 | **0.321***** | **0**.**235*** | **0**.**428***** | **0**.**331***** | 0.182 | -0.151 | 0.096 |
| C24DHC | 0.054 | 0.076 | **0**.**197*** | **0**.**223*** | 0.119 | -**0**.**211*** | 0.104 | -0.123 |
| C24:1DHC | 0.021 | **0.297**** | **0**.**313***** | **0**.**381***** | **0**.**3**** | -0.044 | -0.036 | 0.109 |
| C16Cer | 0.044 | **0.348***** | **0**.**273**** | **0.406***** | **0.347***** | 0.162 | -0.154 | **0**.**257***** |
| C18Cer | 0.134 | 0.18 | 0.168 | **0.465***** | **0.224*** | **0**.**317**** | -0.023 | -0.138 |
| C20Cer | 0.126 | 0.176 | **0**.**243**** | **0**.**436***** | 0.16 | 0.199 | 0.028 | -**0**.**237**** |
| C24Cer | 0.118 | -0.169 | 0.042 | 0.149 | -0.012 | -0.191 | **0**.**259**** | -**0**.**444***** |
| C24:1Cer | 0.104 | 0.16 | **0**.**249**** | **0.417***** | **0.201*** | 0.144 | 0.065 | -0.177 |
| Sphingosine | -0.106 | **0.19*** | **0**.**197*** | **0**.**263**** | **0**.**307**** | 0.084 | 0.113 | 0.091 |
| SIP | -0.151 | -0.134 | 0.003 | 0.146 | 0.135 | -0.041 | **0**.**320***** | -**0**.**375***** |
| SA1P | -0.125 | -0.146 | -0.117 | 0.093 | 0.068 | -0.124 | 0.169 | -**0**.**22*** |

**Correlation is evaluated by Spearman's correlation coefficient rho (r).** Abbreviations: *AST: aspartate aminotransferase, ALT: alanine aminotransferase, γGT: gamma glutamyl transferase, AFP: alpha fetoprotein, CRP: C reactive protein, Hb: hemoglobin, MELD: Mod-el of End stage Liver Disease, DHC: dihydroceramide, Cer: ceramide, SIP: sphingosine 1-phosphate, SA1P: spinganine 1-phosphate.* Significant correlations are shown in bold and are indicated in the corresponding Figures: "*" = p<0.05, "**" = p<0.01, "***" = p<0.001.

### Example 3: Serum C16Cer and SIP show a high diagnostic accuracy in differentiating patients with HCC from cirrhotic patients

Driven from the significant upregulation of SL parameters in the serum of HCC patients, next the diagnostic potential of the above observed associations was evaluated. Since nearly all of the SL's analyzed associated significantly in univariate analysis with the occurrence of HCC, it was intended to conduct a multivariate analysis to evaluate if SL parameters constitute independent diagnostic predictors of HCC. However, a multivariable regression analysis was statistically not feasible since some of the SL parameters, particularly C16Cer and SIP, provided a nearly complete diagnostic separation of HCC patients from patients with liver cirrhosis. Therefore, the diagnostic performance of serum SL's by ROC analysis with the estimation of correspondent AUC was assessed. AUC values, cut-off parameter concentrations and estimation of the wrong classification rate (WCR) are listed Table 3.

**Table 3 AUC values of liver enzymes, MELD score and further biochemical and SL parameters in the prediction of HCC in patients with liver cirrhosis**

| **Parameters** | **AUC ± SD** | **95% CI** | **Cut-off value** | **WCR** | **P-value** |
|---|---|---|---|---|---|
| ALT | 0.742 ± 0.031 | 0.680-0.804 | 35.5 IU/l | 29.1 % | <0.001 |
| AST | 0.732 ± 0.032 | 0.669-0.795 | 63.5 IU/l | 30.9 % | <0.001 |
| γGT | 0.699 ± 0.033 | 0.633-0.764 | 105.5 IU/l | 33.8 % | <0.001 |
| CRP | 0.514 ± 0.041 | 0.433-0.594 | 0.445 mg/l | 45.2% | 0.7 |
| AFP | 0.823 ± 0.028 | 0.766-0.879 | 7.45 ng/ml | 24.1 % | <0.001 |
| Hb | 0.730 ± 0.031 | 0.668-0.793 | 11.35 g/dl | 29.7 % | <0.001 |
| MELD | 0.736 ± 0.031 | 0.675-0.797 | 10.5 | 30.8 % | <0.001 |
| C16DHC | 0.932 ± 0.015 | 0.901-0.963 | 55.2 ng/ml | 13.4% | <0.001 |
| C18DHC | 0.932 ± 0.016 | 0.900-0.965 | 64.2 ng/ml | 11.7 % | <0.001 |
| C24DHC | 0.684 ± 0.033 | 0.618-0.749 | 65.1 ng/ml | 36.3 % | <0.001 |
| C24:1DHC | 0.604 ± 0.035 | 0.533-0.674 | 301 ng/ml | 40.5 % | 0.004 |
| C16Cer | 0.999 ± 0.000 | 0.998-1.000 | 147.5 ng/ml | 1.2% | <0.001 |
| C18Cer | 0.665 ± 0.034 | 0.597-0.732 | 67.7 ng/ml | 36.3 % | <0.001 |
| C20Cer | 0.837 ± 0.025 | 0.788-0.886 | 78.6 ng/ml | 22.1 % | <0.001 |
| C24Cer | 0.826 ± 0.025 | 0.775-0.877 | 953 ng/ml | 23.8 % | <0.001 |
| C24:1Cer | 0.572 ± 0.036 | 0.501-0.643 | 431 ng/ml | 42.6% | 0.048 |
| Sphingosine | 0.759 ± 0.031 | 0.697-0.821 | 4.83 ng/ml | 27.8 % | <0.001 |
| SIP | 0.985 ± 0.005 | 0.974-0.997 | 215 ng/ml | 4.2% | <0.001 |
| SA1P | 0.790 ± 0.029 | 0.733-0.848 | 30.8 ng/ml | 23.8 % | <0.001 |

**The diagnostic performance of serum SL's, assessed by receiver operating characteristic (ROC) curve analysis with the estimation of correspondent areas under the curve (AUC).** Abbreviations: *CI: confidence interval, SD: standard deviation, WCR: wrong classification rate*, *ALT: alanine aminotransferase, AST: aspartate aminotransferase, γGT: gamma-glutamyl*-*transferase*, *AFP: alpha fetoprotein, CRP: C reactive protein, Hb: hemoglobin*, *MELD: Model of End stage Liver Disease, DHC: dihydroceramide, Cer: ceramide, S1P: sphingosine 1-phosphate, SA1P: spinganine 1-phosphate.*

C16Cer and SIP showed the highest AUC values (0.999 and 0.985, P<0.001 respectively) and consequently also the lowest WCR-values (1.2 % and 4.2 % respectively), being thus significantly more accurate than AFP (WCR of 24.1 %) in the differentiation of HCC from liver cirrhosis patients. Moreover, C16DHC and C18DHC appeared also with a high diagnostic accuracy, since their AUC values (for both parameters 0.932, P<0.001) were significantly higher than that of AFP (0.823, P<0.001). Corresponding ROC graphs of serum SL's, AFP and further biochemical parameters are illustrated in Figure 5.

### Example 4: Diagnostic Algorithms

Finally a diagnostic algorithm in the primary diagnosis of HCC and especially early stage BCLC A HCC according to above mentioned cut-off concentrations of C16Cer and SIP was evaluated (Figure 6). AFP, C16Cer and SIP levels were accessible in 106 out of 122 HCC patients and in 22 out of 26 BCLC A HCC patients. Only 33 HCC patients (30.5%) could be diagnostically identified by AFP levels, while C16Cer and SIP were able to identify additional 72 (67.5%) and 70 (64.8%) HCC patients respectively (Figure 6A, 6C). In patients with early stage BCLC A HCC the diagnostic benefit from C16Cer and SIP was even more substantial since additional 20 patients with AFP levels <200 ng/ml could be identified by C16Cer and SIP (Figure 6B, 6D).

In conclusion, this study identifies for the first time significant alterations of SL parameters in the serum of HCC patients and emphasizes the potential of sphingolipidomics in order to query novel biomarkers within the serologic signature of HCC.

## Claims

1. A non-invasive method of predicting, monitoring and/or diagnosing hepatocellular carcinoma (HCC) in a subject, comprising determining the level of one or more biomarkers selected from the group of sphingolipids in a biological sample from the subject.

2. The non-invasive method according to claim 1, comprising the steps of:
(a) Providing the biological sample from the subject,
(b) Determining the level of one or more biomarkers selected from the group of sphingolipids in the biological sample;
(c) Comparing the level of the one or more biomarkers in the biological sample to a control level of the one or more biomarkers;
Wherein a different level of the one or more biomarkers in the biological sample compared to the control level is indicative for the presence or absence of HCC in the subject.

3. The method according to claim 1 or 2, wherein the one or more biomarkers is selected from a long chain ceramide, or a long chain dihydroceramide.

4. The method according to any of claims 1 to 3, wherein the one or more biomarkers is selected from a C16 to C24 Ceramide or a C16 to C24 dihydroceramide.

5. The method according to any of claims 1 to 4, wherein the one or more biomarkers is selected from the group C16Cer, C16DHC, C18DHC, and SIP, and preferably is C16Cer.

6. The method according to any of claims 1 to 5, wherein the biological sample is a blood sample, or blood derived sample.

7. The method according to any of claims 1 to 6, wherein diagnosing HCC comprises diagnosing in a subject having liver cirrhosis the presence or absence of HCC.

8. The method according to claim any one of claims 1 to 7, wherein the control level is a cut-off level, and an increased level or equal level of the one or more biomarkers in the biological sample compared to the cut-off level is indicative for the presence HCC.

9. The method according to any of the preceding claims, wherein the subject is a mammal, preferably a human.

10. The method according to any of the preceding claims, wherein determining the level of the one or more biomarkers is performed using chromatography, mass spectrometry, or immunological methods, such as antibody based methods.

11. A non-invasive method for monitoring a treatment of a liver disease in a subject, the method comprising
(a) Providing a first biological sample of the subject that was taken at a first time point of the therapy,
(b) Providing a second biological sample of the subject that was taken at a second time point of the therapy, wherein the second time point is later that the first time point,
(c) Determining the level of one or more biomarkers selected from the group of sphingolipids in the first and the second biological sample,
(d) Comparing the level of the one or more biomarkers in the first biological sample with the level of the one or more biomarkers in the second biological sample,
Wherein an increase of the level of the one or more biomarkers in the second biological sample compared to the first biological sample is indicative for a treatment success.

12. The method according to claim 11, wherein the one or more biomarkers is selected from the group of C16Cer, C16DHC, C18DHC, SIP, C24DHC, C24:1DHC, C18Cer, C20Cer, C24Cer, C24:1Cer, Sphingosine, and SA1P.

13. The method according to claim 11 or 12, wherein the liver disease is HCC.

14. A diagnostic kit for performing a method according to any of claims 1 to 13, wherein the kit comprises means for determining the level of the one or more biomarkers in the biological sample.
